# EUROPÄISCHE PATENTANMELDUNG

(11) **EP 1 090 906 A1**
(43) Veröffentlichungstag der Anmeldung: **11.04.2001**
(21) Anmeldenummer: 01100055.1
(22) Anmeldetag: 23.07.1996
(51) Int. Cl.: C07C 211/50, C07C 263/10, C07C 209/36

(54) **Gemische aus Toluylendiamin und Wasser**

(30) Priorität: 04.08.1995 DE 19528781
(62) Teilanmeldung aus: 96111809.8
(71) Anmelder: BAYER AG, 51368 Leverkusen (DE)
(72) Erfinder: Biskup, Klaus, Dr., 51373 Leverkusen (DE); Keggenhoff, Berthold, Dr., 47839 Krefeld (DE)

(57) **Zusammenfassung**

Toluylendiisocyanat wird durch Nitrierung von Toluol zu Dinitrotoluol, Hydrierung des Dinitrotoluols, gegebenenfalls in Anwesenheit eines Lösungs- oder Verdünnungsmittels, zu einer Rohlösung aus Toluylendiamin und Reaktionswasser, Aufarbeitung der Toluylendiamin-Rohlösung zu trockenem Toluylendiamin von kommerzieller Qualität und Phosgenierung des Toluylendiamins zu Toluylendiisocyanat hergestellt, wobei die destillative Aufarbeitung der Toluylendiamin-Rohlösung zum wasserfreien Toluylendiamin, gegebenenfalls nach vollständiger oder partieller Abtrennung eines verwendeten Lösungs- oder Verdünnungsmittels in einer ersten Produktionsstätte bei einem Wassergehalt von 1-40 Gew.-%, bevorzugt 2-10 Gew.-%, unterbrochen wird, das so erhaltene Gemisch in flüssiger Form zu einer anderen Produktionsstätte transportiert wird, wo die destillative Trocknung des Toluylendiamins vollendet wird, gegebenenfalls weitere Aufarbeitungsstufen angeschlossen werden und danach das Toluylendiamin in an sich bekannter Weise zum Toluylendiisocyanat phosgeniert wird.

Die Erfindung betrifft ferner Gemische aus Toluylendiamin und Wasser mit einem Erstarrungspunkt von höchstens 95°C und deren Verwendung zur Herstellung von Toluylendiisocyanat, die vorzugsweise an einem Ort erfolgt, der vom Ort der Herstellung der Gemische aus Toluylendiamin und Wasser unterschiedlich ist.

## Beschreibung

Die vorliegende Erfindung betrifft ein Verfahren zur Herstellung von Toluylendiisocyanat (TDI) durch Umsetzung von Toluol mit Salpetersäure, Hydrierung des erhaltenen Dinitrotoluols (DNT) zum Toluylendiamin (TDA) und Umsetzung des Amins mit Phosgen zum TDI, wobei die Verfahrensschritte bis zum TDA in einer ersten Produktionsstätte und die Umsetzung des TDA zum TDI in einer weit entfernt gelegenen zweiten Produktionsstätte ausgeführt werden und das TDA in Form einer wässrigen Rohlösung von der ersten Produktionsstätte zur anderen transportiert wird.

Die Erfindung betrifft ferner spezielle Gemische aus Toluylendiamin und Wasser und deren Verwendung zur Herstellung von Toluylendiisocyanat an einem Ort, der unterschiedlich von dem Ort ist, an dem die Gemische aus Toluylendiamin und Wasser erhalten bzw. hergestellt worden sind.

Normalerweise wird die großtechnische Herstellung von TDI mit den Verfahrensstufen Umsetzung von Toluol mit Salpetersäure zu DNT und Wasser, Umsetzung des DNT mit Wasserstoff zu TDA und Wasser und schließlich Umsetzung des aufgearbeiteten, trocknen TDA mit Phosgen zu TDI und Chlorwasserstoff in miteinander verbundenen Produktionsanlagen an einer Produktionsstätte durchgeführt.

Es kann aber vorteilhaft sein, die Verfahrensschritte an zwei weit voneinander entfernten Produktionsstätten durchzuführen, wobei an einer Produktionsstätte die Verfahrensstufen bis zum TDA stattfinden und an der zweiten Produktionsstätte die Umsetzung des TDA mit Phosgen sowie die Aufarbeitung zum verkaufsfähigen TDI-Endprodukt durchgeführt werden. Eine solche Verfahrensweise kann wirtschaftlich attraktiv sein, wenn z.B. in einem Land günstige Rohstoff- und Infrastrukturvoraussetzungen gegeben sind, in einem anderen weit entfernten Land aber große Abnehmermärkte mit dem Zwang zu einer lokalen Produktion des Endprodukts gegeben sind. Ferner kann es wirtschaftlich vorteilhaft sein, verschiedene kleinere Phosgenieranlagen aus einer zentralen, tief rückwärts integrierten Aminanlage zu versorgen.

Diese Arbeitsweise wird aber erheblich dadurch erschwert, dass das Zwischenprodukt TDA einen hohen Schmelzpunkt hat, der den Transport nur entweder in fester Form oder als heiße Schmelze bei über 100°C zulässt. Beim Transport in fester Form muss zunächst eine aufwendige Konfektionierung des TDA, z.B. in Schuppen, vorgenommen werden, um nach dem Transport das Produkt für die Endumsetzung wieder aufschmelzen zu können. Andererseits erfordert der Überseetransport als heiße Schmelze den Einsatz beheizbarer Tankcontainer und entsprechender Beheizungsmöglichkeiten an Bord des Schiffes, z.B. Anschlüsse für Heizdampf oder für elektrischen Strom, oder - im Fall des Transports als Massengut - den Einsatz von Tankschiffen, deren Ladetanks auf eine Temperatur von 105-110°C beheizbar sind. Der erste Fall - Einsatz beheizbarer Tankcontainer - ist sehr kostspielig und für den Transport großer Mengen unwirtschaftlich, der zweite Fall - Transport als Massengut in Schiffen mit heizbaren Ladetanks - wäre ebenfalls sehr teuer und damit unwirtschaftlich, da die üblichen Tankschiffe und auch die Umschlageinrichtungen in den Häfen nicht für die hohen Temperaturen vorgesehen sind und erst mit viel Geld dafür eingerichtet werden müssten. Schließlich wäre das Problem der Entsorgung des Slops aus den Schifftanks, gegebenenfalls durch teure Sondermüllverbrennung an Land, zu bewältigen.

Aufgabe der vorliegenden Erfindung war es, TDA-Formulierungen zur Verfügung zu stellen, welche sich in flüssiger Form bei einer Temperatur unterhalb 95°C ohne Feststoffabscheidung lagern oder transportieren lassen.

Eine weitere Aufgabe bestand darin, das Verfahren für die Herstellung von TDI durch Nitrierung von Toluol zu DNT, Hydrieren des DNT zu TDA und Wasser und Umsetzen des getrockneten TDA mit Phosgen zum TDI so zu modifizieren, dass es die Möglichkeit zu einem Transport des TDA in Tankcontainern oder als Massengut von einer ersten Produktionsstätte zu einer zweiten, weit entfernt gelegenen Produktionsstätte ohne die genannten Nachteile bietet.

Diese Aufgaben werden durch die erfindungsgemäßen Gemische und Verfahren gelöst.

Überraschenderweise hat sich nun gezeigt, dass bestimmte TDA-Wassermischungen mit einem Wassergehalt von 1-40 %, bevorzugt 2-10 %, und gegebenenfalls herstellungsbedingten anderen Nebenprodukten deutlich erniedrigte Schmelzpunkte haben und dadurch wesentlich leichter handhabbar und kostengünstiger als Massengut in Tankschiffen über weite Entfernungen zu transportieren sind als das reine TDA. Die für den Chemikalientransport eingerichteten Tankschiffe können üblicherweise das Ladegut bis zu dem Temperaturbereich von 65 bis 70°C während des Transports temperieren und bei dieser Temperatur auch umschlagen. Ziel des erfindungsgemäßen Verfahrens ist daher, TDA-Wassermischungen zu erhalten, deren Schmelzverhalten den Transport als Massengut in üblichen Tankschiffen ermöglicht.

Solche erfindungsgemäßen Gemische können im Prinzip durch Vermischen von reinem TDA, d.h. dem technisch üblichen Isomerengemisch von ca. 80 Gew.-% 2.4-TDA und ca. 20 Gew.-% 2.6-TDA, mit Wasser erzeugt werden. Sie sind aber einfacher zugänglich, wenn man den technischen Prozess zur Herstellung von TDA durch Hydrierung von DNT an geeigneter Stelle der Aufarbeitung zu trockenem TDA unterbricht. Durch die dann noch enthaltenen Nebenprodukte wie die 2,3- und 3,4-TDA-Isomeren und höhermolekulare Nebenprodukte wird eine weitere Absenkung des Schmelzpunktes erreicht.

Gegenstand der Erfindung ist somit ein Verfahren zur Herstellung von Toluylendiisocyanat durch Nitrierung von Toluol zu Dinitrotoluol, Hydrierung des Dinitrotoluols, gegebenenfalls in Anwesenheit eines Lösungs- oder Verdünnungsmittels, zu einer Rohlösung aus Toluylendiamin und Reaktionswasser, Aufarbeitung der Toluylendiamin-Rohlösung zu trockenem Toluylendiamin von kommerzieller Qualität und Phosgenierung des Toluylendiamins zu Toluylendiisocyant, dadurch gekennzeichnet, dass die destillative Aufarbeitung der Toluylendiamin-Rohlösung zum wasserfreien Toluylendiamin, gegebenenfalls nach vollständiger oder partieller Abtrennung eines verwendeten Lösungs- oder Verdünnungsmittels, in einer ersten Produktionsstätte bei einem Wassergehalt von 1-40 Gew.-%, bevorzugt 2-10 Gew.-%, unterbrochen wird, das so erhaltene Gemisch zu einer anderen Produktionsstätte transportiert wird, wo die destillative Trocknung des Toluylendiamins vollendet wird, gegebenenfalls weitere Aufarbeitungsstufen angeschlossen werden und danach das Toluylendiamin in an sich bekannter Weise zum Toluylendiisocyanat phosgeniert wird.

Erfindungsgemäß bevorzugt ist,
- dass als Toluylendiamin 2.4- oder 2.6-Toluylendiamin oder beliebige Gemische dieser Isomeren, gegebenenfalls mit unterschiedlichen Gehalten an 2.3- und 3.4-Isomeren, zum Einsatz kommen, und
- dass bei der Hydrierung des Dinitrotoluols ohne Lösungs- oder Verdünnungsmittel gearbeitet wird.

Die Erfindung betrifft auch Gemische aus Toluylendiamin und Wasser und gegebenenfalls herstellungsbedingten anderen Nebenprodukten mit einem Erstarrungspunkt von höchstens 95°C.

Erfindungsgemäß bevorzugt sind solche Gemische mit einem Erstarrungspunkt im Bereich von 60-95°C, vorzugsweise 65-70°C,
- die aus 2.4- und/oder 2.6-Toluylendiamin, gegebenenfalls weiteren Isomeren des Tolylendiamin sowie höhermolekulare aminische Komponenten in geringeren Anteilen und Wasser bestehen,
- die auf 100 Gew.-Teile Toluylendiamin 1-40 Gew.-Teile Wasser enthalten und - die auf 100 Gew.-Teile Toluylendiamin 2-10 Gew.-Teile Wasser enthalten.

Neben dem angegebenen Wassergehalt können die erfindungsgemäßen Gemische gegebenenfalls einen Gehalt an organischen homogen gelösten Lösungs- oder Verdünnungsmitteln von max. 10 Gew.-% enthalten. Geeignete Lösungsmittel oder Verdünnungsmittel sind dabei insbesondere niedere Alkohole, vorzugsweise Methanol, Ethanol, n- und Isopropanol, niedere Ketone, insbesondere Aceton, sowie Diole, insbesondere Ethylenglykol, oder auch Toluol.

Gegenstand der Erfindung ist ferner die Verwendung der Gemische zur Herstellung von Toluylendiisocyanat durch Phosgenierung unter vorheriger Entfernung des Wassers, gegebenenfalls enthaltener Lösungsmittelanteile sowie bevorzugt auch der Nebenkomponenten wie z.B. der 2,3- sowie 3,4-TDA-Isomeren.

Bevorzugt ist dabei, dass die Gemische zwecks Herstellung von Toluylendiisocyanat zu einer Produktionsstätte transportiert werden, die vom Ort der Herstellung der Gemische gemäß Anspruch 4 bis 8 räumlich entfernt ist.

Die technische Herstellung von TDA aus Dinitrotoluol erfolgt im allgemeinen im kontinuierlichen Verfahren durch Reduktion der Nitrogruppen des DNT mit Wasserstoff unter erhöhtem Druck an einem suspendierten, pulverförmigen Katalysator, z.B. Palladium auf Aktivkohle oder Raney-Nickel, gegebenenfalls unter Verwendung eines Verdünnungs- oder Lösungsmittels. Verfahren zur Herstellung von aromatischen Aminen wie TDA durch katalytische Hydrierung der entsprechenden Nitroverbindungen, hier DNT, sind in großer Zahl bekannt geworden (vgl. z.B. DE-OSen 1 542 544, 1 947 851, 2 016 644, 2 135 154, 2214056, 2456308, BE-PSen 631 964, 661 047, 661 946, FR-PS 1 359 438, GB-PS 768 111, EP-A 0 124 010).

Die Verwendung von Lösungs- oder Verdünnungsmitteln, z.B. Methanol, Ethanol oder Propanol (Ullmann, 4. Aufl. 1977, Bd. 13, S. 14) dient dazu, die hohe Reaktionswärme von ca. 418 kJ/mol Nitrogruppe in einer größeren Menge Reaktionsgut zu verteilen und ihre Abfuhr zu erleichtern, sowie die Verfügbarkeit des Dinitrotoluols in der Suspension durch Verbesserung der Löslichkeit zu erhöhen. Diesen wünschenswerten Eigenschaften zuwiderlaufend ist die Verwendung von Lösungsmitteln mit zusätzlichen Destillationskosten für ihre Abtrennung verbunden, so dass die Minimierung des Lösungsmitteleinsatzes unter gleichzeitiger Beachtung der sicherheitstechnischen und reaktionstechnischen Erfordernisse ein Optimierungsziel darstellt. Bevorzugt für das erfindungsgemäße Verfahren ist daher die kontinuierliche Herstellung von TDA ohne Lösungsmittel, wie sie in Reaktoren nach DE-OS 36 35 217 möglich ist, die konstruktiv so gestaltet sind, dass sie durch Verdampfungskühlung, d.h. durch Erzeugung von z.B. Wasserdampf auf der Kühlmittelseite, für eine besonders leistungsfähige Wärmeabfuhr sorgen und damit im Fall des Dinitrotoluols die Durchführung der Reaktion bei höherer Temperatur als sonst üblich - nämlich 180-200°C statt 100-150°C - und ohne Lösungsmittel gestatten.

Die, bei der Umsetzung von Dinitrotoluol mit Wasserstoff erhaltene Rohlösung von ca. 60 Gew.-% TDA mit ca. 40 Gew.-% Reaktionswasser und den gegebenenfalls verwendeten Verdünnungs- oder Lösungsmitteln, enthält nach Abtrennung des festen Katalysators - z.B. durch Filtration oder Sedimentation - außerdem die Nebenprodukte des Prozesses, vor allem ca. 3-5 Gew.-% des 2.3- und des 3.4-TDA-Isomeren, aber auch sonstige Nebenprodukte, z.T. mit höherem Molekulargewicht. Zur Herstellung eines für die Phosgenierung verwendbaren Ausgangsproduktes müssen Wasser und gegebenenfalls verwendetes Verdünnungs- oder Lösungsmittel vollständig, z.B. destillativ, in an sich bekannter Weise abgetrennt werden. Es ist außerdem zur Erzielung einer möglichst hohen Ausbeute an TDI zweckmäßig, auch die Nebenprodukte destillativ abzutrennen, wodurch die Reinheit des kommerziell üblichen TDA erzielt wird.

Vor der Abtrennung des gebildeten Reaktionswassers muss die rohe, vom Katalysator befreite Reaktionslösung gegebenenfalls zuerst von dem verwendeten Lösungsmittel befreit werden. Dies geschieht im allgemeinen in bekannter Art durch Destillation in einer kontinuierlich betriebenen Destillationskolonne, wobei das Lösungsmittel durch geeignete Verfahrensführung so rein zurückerhalten wird, dass es ohne weitere Reinigung direkt wieder im Prozess verwendet werden kann. Es ist auch möglich, das verwendete Lösungsmittel gemeinsam mit einem Teil oder der gesamten Menge des gebildeten Wassers aus der TDA-Rohlösung destillativ abzutrennen und das Lösungsmittel daran anschließend in einer weiteren Verfahrensstufe in der benötigten Reinheit zurückzugewinnen. Bei Durchführung der Reaktion ohne Lösungsmittelzusatz ist diese Lösungsmittelabtrennung selbstverständlich nicht nötig.

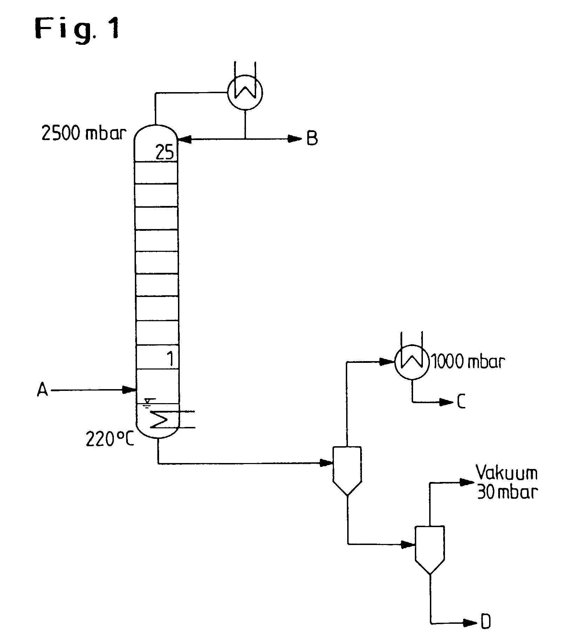

Es schließt sich dann üblicherweise die eigentliche TDA-Trocknung an, d.h. die restlose Entfernung des Reaktionswassers, das ca. 40 Gew.-% von der rohen TDA-Lösung ausmacht. Dies kann prinzipiell durch einfaches Abtreiben des Wassers durch Erhitzen der TDA-Lösung im Vakuum und Abziehen der gebildeten Dämpfe erfolgen. Jedoch fällt bei dieser einfachen Verfahrensführung das abgetrennte Wasser nicht in der für eine problemlose Entsorgung benötigten Reinheit an, sondern ist stets mehr oder weniger mit TDA verunreinigt. Das Reaktionswasser wird daher besser durch Destillation in einer geeigneten Destillationsapparatur entfernt. Dazu wird z.B. die TDA-Rohlösung in einer Kolonne bis auf eine Temperatur von mehr als 200°C im Sumpf erhitzt, wobei das Wasser am Kopf in reiner Form anfällt, wenn die Kolonne bei Normaldruck oder im leichten Überdruckbereich betrieben wird und etwa 20 bis 30 Böden besitzt, und das TDA aus dem Sumpf abgezogen und durch Entspannung ins Vakuum von 30 bis 50 mbar von letzten Wasserspuren befreit wird. Fig. 1 zeigt das herkömmliche Verfahren schematisch.

In Abweichung zu dem bis jetzt dargestellten üblichen Verfahrensablauf der TDA-Herstellung wird im erfindungsgemäßen Verfahren die Wasserabtrennung in zwei Schritten durchgeführt. Zwar hat die rohe TDA-Lösung mit ca. 40 Gew.-% Wasseranteil den sehr niedrigen Erstarrungspunkt von etwa 45°C und wäre damit direkt für den Massenguttransport in Tankschiffen geeignet. Unter Erstarrungspunkt wird dabei die Temperatur verstanden, bei der der Übergang vom flüssigen in den festen Zustand erfolgt. Doch würde dies bedeuten, dass man teuren Laderaum in erheblichem Maße für den Transport von Wasser verschwenden würde. Im erfindungsgemäßen Verfahren wird daher eine Teilentfemung des Reaktionswassers aus der rohen TDA-Lösung vorgenommen, wobei die Restwasserkonzentration im TDA so eingestellt wird, dass der Erstarrungspunkt der resultierenden Lösung nicht oberhalb des auf Tankschiffen üblicherweise beherrschten Temperaturbereiches liegt. Als Grundlage dafür dient die im Diagramm 1 dargestellte Abhängigkeit des Erstarrungspunktes der TDA-Rohlösung vom Wassergehalt. Danach entspricht z.B. der Erstarrungspunkt von 65°C einem Wassergehalt von 7 Gew.-%.

Das für die Teilentfemung des Wassers aus der rohen TDA-Lösung durchgeführte erfindungsgemäße Verfahren ist sehr viel einfacher als die oben beschriebene Apparatur zur Trocknung des TDA. Bevorzugt wird dazu eine einfache Normaldruck-Destillation durchgeführt, wobei zur Herstellung des TDA-freien Kopfprodukts eine Kolonne mit nur 5 praktischen Böden vollständig ausreicht. Da auf diese Weise die Hauptmenge des Wassers bereits abgetrennt wird, kann die für die Feintrocknung am Zielort benötigte Vakuumdestillationsapparatur mit ca. 10 praktischen Böden kleiner als sonst üblich und damit preiswerter sein. Die Auslegung der Kolonnen erfolgt in an sich bekannter Weise durch Berücksichtigung des Phasengleichgewichtes für das System TDA/Wasser (Diagramm 2).

Ein weiterer Vorteil des erfindungsgemäßen Verfahrens ist die problemlose Entsorgung des Slops aus den Schifftanks. Da die Schiffsspülung mit Wasser durchgeführt werden kann und an jedem Zielort die destillative Aufarbeitung von wässrigem TDA ausgeführt wird, ist dort auch die Aufarbeitung der TDA-haltigen Spülwasser möglich und teure Sondermüllentsorgungen sind nicht notwendig.

Die weitere Verarbeitung des getrockneten TDA zum TDI am Zielort geschieht in der auch sonst üblichen Verfahrensweise. Gegebenenfalls erfolgt zunächst aus dem TDA-Isomerengemisch die Abtrennung der 2.3- und 3.4-Isomeren (US 3 420 752, US 3 414 619) und sonstiger Nebenprodukte. Das Verfahren der TDI-Herstellung durch Phosgenierung des TDA läuft dann in bekannter Weise ab, vgl. z.B. Becker/Braun, Kunststoff-Handbuch, 2. Auflage, 1983, Bd. 7, S. 63 ff, Carl-Hanser Verlag, München, und dort zitierter Literatur.

Gegenstand der vorliegenden Erfindung ist also ein Verfahren zur Herstellung von TDI durch Nitrierung von Toluol zu Dinitrotoluol (DNT), Hydrierung des DNT zu einer Rohlösung aus Toluylendiamin (TDA) und Reaktionswasser, gegebenenfalls in Anwesenheit eines Lösungs- oder Verdünnungsmittels, bevorzugt jedoch ohne Verwendung eines solchen, Aufarbeitung der TDA-Rohlösung zu trocknem TDA von kommerzieller Qualität und Phosgenierung zu TDI, wobei die Aufarbeitung der TDA-Rohlösung - gegebenenfalls nach vollständiger oder partieller Abtrennung eines verwendeten Lösungsmittels - in einer ersten Produktionsstätte bei Erzielen eines Wassergehaltes von 1 bis 40 Gew.-%, bevorzugt 2 bis 10 Gew.-%, unterbrochen wird, das so erhaltene Gemisch zu einer zweiten Produktionsstätte transportiert wird, dort die destillative Trocknung des TDA vollendet und gegebenenfalls weitere Aufarbeitungsstufen, beispielsweise die Abtrennung der 2.3- und 3.4-TDA-Isomeren angeschlossen werden.

Geeignet für das erfindungsgemäße Verfahren sind 2.4-Toluylendiamin sowie Isomerengemische des Toluylendiamin, typischerweise 2.4/2.6-Toluylendiamin-Gemische der Isomerenverhältnisse 65/35 und 80/20, gegebenenfalls mit unterschiedlichen Gehalten an 2.3- und 3.4-Isomeren typisch 0,05 bis 5,0 Gew.-%, sowie mit unterschiedlichen Anteilen an höhermolekularen Nebenprodukten, typisch 0,01 bis 2,5 Gew.-%, üblich 0,2 bis 1,8 Gew.-%.

Die nachfolgenden Beispiele sollen die Erfindung näher erläutern, ohne sie jedoch in ihrem Umfang zu begrenzen.

### Beispiele

### Beispiel 1

### Herstellung eines erfindungsgemäßen TDA/Wasser-Gemisches

### a) Herstellung von Dinitrotoluol

Die Herstellung des Dinitrotoluols (DNT) erfolgte in zwei mit Wasser gekühlten Rührkesselreaktoren von je 5001 Volumen mit seitlichem Überlauf in Trennflaschen von je 100 1 Volumen. Entlüftungsseitig war die Apparatur an eine Abgassammelleitung angeschlossen, die bei Normaldruck betrieben wurde. Vor dem Anfahren der Reaktion wurde die Apparatur mit 92 %iger Schwefelsäure gefüllt und die Rührer wurden gestartet.

In den ersten Kessel wurden kontinuierlich 93 kg/h Toluol und 100 kg/h 65 %ige Salpetersäure eingespeist sowie die Schwefelsäure, die als 92 %ige Säure in den zweiten Kessel mit 665 kg/h eingetragen worden und in die nachgeschaltete Scheideflasche übergelaufen war.

Der Überlauf des ersten Kessels trennte sich in der zugehörigen Scheideflasche in eine Schwefelsäurephase, die zur Aufarbeitung ausgeschleust wurde, und in eine organische Phase, die überwiegend aus Mononitrotoluol (MNT) bestand und kontinuierlich in den zweiten Kessel gepumpt wurde. Dort wurden außer der obengenannten 92 %igen Schwefelsäure noch 105 kg/h 65 %ige Salpetersäure zugegeben. Die Temperatur im zweiten Kessel betrug 70°C.

Der Überlauf des zweiten Kessels trennte sich in der zugehörigen Scheideflasche in die obengenannte Schwefelsäure, die in den ersten Kessel gepumpt wurde, und in die organische Phase, die das rohe DNT und anhaftende Säurespuren enthielt.

Zur Reinigung durchlief das rohe DNT kontinuierlich eine auf 70°C temperierte 3-stufige Mischer/Settler-Batterie, in der es nacheinander mit 50 l/h warmem Wasser (70°C), 50 l/h 2 %ige Natronlauge (70°C) und 50 l/h entionisiertem Wasser (70°C) gewaschen wurde. Ein wässriger Auszug des resultierenden DNT hatte den pH-Wert 7.6. Die wässrigen Phasen wurden als Abwasser verworfen.

### b) Hydrierung des DNT zum TDA

Zur Hydrierung des DNT zum Toluylendiamin diente ein 5001 Autoklav mit Kühl- und Heizmöglichkeit, Begasungsrührer, Thermometer und Standmessung.

In dem Autoklav wurde ein Gemisch aus 70 kg Wasser und 150 kg Toluylendiamin bei 80°C vorgelegt; es wurden ca. 7 kg Raney-Nickel, suspendiert in ca. 301 Wasser, zugegeben. Der Autoklav wurde 10 Mal mit 10 bar Wasserstoff bespannt und jeweils wieder entspannt. Dann wurden 22 bar Wasserstoff aufgedrückt, der Rührer eingeschaltet und mit der Zugabe von 182 kg/h DNT über ein Tauchrohr in das Reaktionsgemisch begonnen.

Nach etwa 2 Minuten wurde das Anspringen der Reaktion durch rasches Ansteigen der Autoklaveninnentemperatur erkennbar. Nach Umschalten der Beheizung auf Kühlung wurde die Temperatur auf 190°C eingeregelt. Der Verbrauch an Wasserstoff wurde durch Zugabe von frischem Wasserstoff ausgeglichen, so dass der Druck im Autoklaven konstant 22 bar betrug. Aus dem Gasraum des Autoklaven wurde ein Gasstrom von 25 Nm³/h ausgeschleust.

Als der Stand im Autoklaven 75 % erreichte, wurde mit der Produktentnahme über ein Tauchrohr begonnen. Das Produkt strömte in eine Filtrationsvorlage von ca. 201 Inhalt, in die eine Sintermetall-Filterkerze 3 µ eingesetzt war.

Mit einer Pumpe wurden aus der Filtrationsvorlage ca. 50 l/h mit Katalysator angereichertes Reaktionsgemisch wieder in den Reaktor zurückbefördert, während das durch die Filterkerze geströmte Reaktionsgemisch über ein Regelventil, das vom Stand im Reaktor geregelt wurde, in einen Entspannungsbehälter mit Rückflusskühler auf 3 bar entspannt wurde. Die entspannten Gase wurden als Abluft abgegeben.

Aus dem Entspannungsbehälter floss das Reaktionsgemisch kontinuierlich in eine erste Destillationskolonne mit 5 Glockenböden, die eine Länge von ca. 2,5 m und einen Durchmesser von 200 mm hatte. Die Kolonne wurde unter Umgebungsdruck betrieben und im Sumpf mit einem Einsteckverdampfer mit 6 bar Dampf auf 135°C beheizt. Am Kopfkondensator wurde bei einem Rücklaufverhältnis R:E = 3 Wasser mit 25 ppm TDA abgenommen. Das erfindungsgemäße TDA/Wasser-Gemisch war das Sumpfprodukt und enthielt 7 Gew.-% Wasser bei einem Erstarrungspunkt von 65°C. Es zeigte nach 4 Wochen Lagerung bei 70°C keine Feststoffausscheidung und war somit für einen Schiffs-Bulktransport bei 70°C geeignet.

### Beispiel 2

### Herstellung von Toluylendiisocyanat aus einem erfindungsgemäßen TDA/Wasser-Gemisch

In einer zweiten Destillationskolonne mit 10 Glockenböden, die ca. 4,5 m lang war und einen Durchmesser von 300 mm hatte, wurde das Produkt aus der ersten Kolonne im Vakuum bei 100 mbar vom Restwasser befreit. Die Kolonne wurde im Sumpf mit 30 bar Dampf beheizt; das TDA hatte einen Wassergehalt von 400 ppm, das Kopfprodukt Wasser hatte bei R:E = 4 einen TDA-Gehalt von 10 ppm.

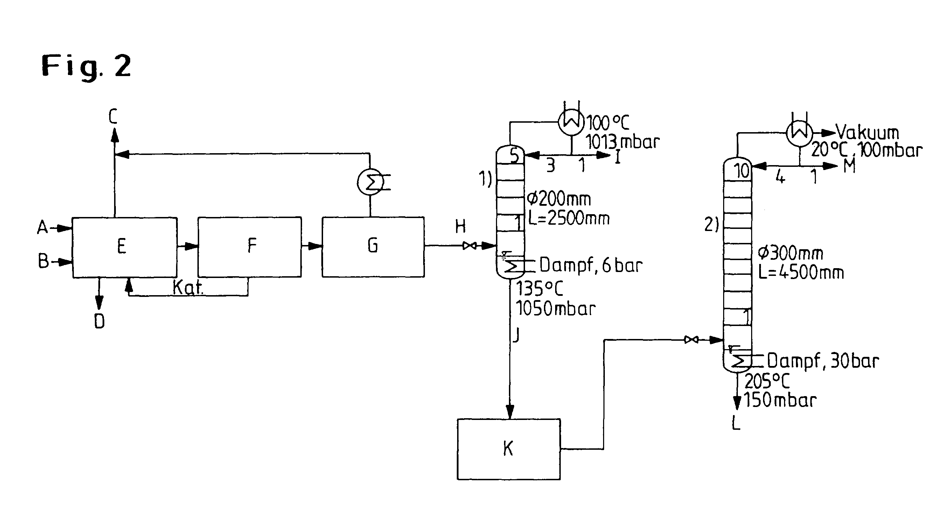

In Fig. 2 ist die Destillation schematisch dargestellt.

Die Umsetzung des TDA mit Phosgen zum TDI erfolgte in einer kontinuierlich betriebenen Apparatur, die im wesentlichen aus zwei mit 30 bar Dampf beheizten Rührkesseln mit je 2 m³ Inhalt und zwei Destillationskolonnen bestand.

In den ersten der beiden Rührkessel wurden 800 kg/h Phosgen in Form einer 50 %igen Lösung in Orthodichlorbenzol (ODB) eingespeist und dort bei einem Druck von 1,9 bar auf 90°C erhitzt. Der Überlauf floss dem zweiten Rührkessel zu, der entlüftungsseitig mit dem ersten verbunden war und auf 135°C gehalten wurde.

Das TDA, das mit 120 kg/h mit 45°C in Form einer 5 %igen Lösung in ODB mit einer Pumpe aus einem TDA-Lösungstank entnommen wurde, wurde der Phosgenlösung, die in den ersten Rührkessel eingeleitet wurde, zugemischt. Zur intensiven Durchmischung der beiden Lösungen diente eine Kreiselpumpe mit offenem Laufrad.

Das aus dem zweiten Rührkessel austretende Reaktionsgemisch wurde in einem mit 30 bar Dampf betriebenen Wärmetauscher auf 190°C erhitzt.

Die aus den Rührkesseln und dem Wärmetauscher austretenden Dämpfe wurden durch eine Auswaschkolonne mit einem Durchmesser von 500 mm, einer Länge von ca. 4 m und mit 10 Glockenböden geleitet. Ein mit gekühltem ODB bei 60°C betriebener Kondensator am Kopf der Kolonne kondensierte einen Teil des Reaktionsgemisches, das als Rücklauf auf die Kolonne gegeben wurde. Der Ablauf der Kolonne wurde getaucht in den ersten Rührkessel zurückgeführt. Die aus dem Kondensator austretenden Dämpfe wurden zur Trennung des im Überschuss eingesetzten Phosgens von dem erzeugten Chlorwasserstoff einer Rückgewinnungsapparatur zugeleitet.

Das mit 190°C aus obengenanntem Wärmetauscher austretende Reaktonsgemisch wurde in den Sumpf der Lösungsmittelkolonne, die einen Durchmesser von 450 mm, eine - Länge von ca. 6 m und eine Füllkörperpackung von ca. 4 m Höhe besaß, eingeleitet. Der Kolonnensumpf wurde mit einem Einsteckdampfer mit 30 bar Dampf auf ca. 185°C erhitzt, der Druck am Kopf der Kolonne betrug 330 mbar. Mit einem mit Kühlwasser betriebenen Kondensator wurde ein Rücklaufverhältnis von R:E = 2 eingestellt. Das ausgeschleuste ODB enthielt kein TDI und wurde zur Herstellung der TDA-Lösung und der Phosgenlösung zurückgeführt.

Das dem Sumpf der Lösungsmittelkolonne entnommene TDI mit etwa 10 % ODB wurde in einem nachgeschalteten Dünnschichtverdampfer (DSV), der mit 30 bar Dampf beheizt wurde, und unter dem Druck von 100 mbar stand, vollständig vom ODB befreit. Das DSV-Destillat wurde in einem Kondensator mit Kühlwasser kondensiert und in die Lösungsmittelkolonne zurückgegeben.

Der DSV-Sumpfaustrag wurde in einem zweiten Dünnschichtverdampfer, der ebenfalls mit 30 bar Dampf beheizt wurde und unter einem Vakuum von 10 mbar betrieben wurde, in ca. 90 % Kopfprodukt und ca. 10 % Sumpfabzug aufgeteilt. Der Sumpfabzug wurde in einer Destillationsblase von 2 m³ Volumen gesammelt und diskontinuierlich mit 30 bar Dampf bei 220°C/5 mbar bis zur zähen Schmelze eingeengt, die heiß abgelassen anschließend in Pappkübeln erstarrte und über eine Abfallverbrennung entsorgt wurde.

Das Destillat der Blasendestillation wurde gemeinsam mit dem Destillat des zweiten DSV in die TDI-Kolonne eingespeist.

Die TDI-Kolonne hatte eine Gesamthöhe von ca. 5 m, einen Durchmesser von 250 mm und eine Füllkörperpackung von etwa 3,5 m Länge. Am Kopf der Kolonne befand sich ein Kondensator, der totalen Rücklauf erzeugte, die Produktentnahme geschah unterhalb des obersten Füllkörperschusses von ca. 400 mm mittels eines Entnahmebodens. Das Rücklaufverhältnis R:E war 15. Am Kopf der Kolonne herrschte ein Druck von 15 mbar.

Der Sumpf der TDI-Kolonne hatte ein Volumen von ca. 1001 und wurde mittels eines Einsteckverdampfers mit 30 bar Dampf beheizt, so dass die Temperatur 163°C betrug.

Der Sumpfstand wurde im Abstand von 6 h jeweils um ca. 301 abgesenkt, das ausgeschleuste Produkt wurde dem Zulauf des obengenannten zweiten DSV zugegeben.

Das entnommene TDI-Produkt zeigte die folgenden analytischen Werte:

| | |
|---|---|
| 79,4 % | 2.4-Isomeres, |
| 20,6 % | 2.6-Isomeres, |
| 0,005 % | Hydrolisierbares Chlor |

und weniger als 0,005 % ODB. Es entsprach damit der Qualität des handelsüblichen TDI.

Die Ausbeute über alle Fertigungsstufen vom Toluol bis zum Toluylendiisocyanat betrug ca. 85 %.

### Erläuterung der Figuren:

**Fig. 1**: Herkömmliche Wasserabtrennung aus TDA-Rohlösungen nach dem Stand der Technik. Es bedeuten:

| | |
|---|---|
| A | TDA/Wasser-Gemisch mit ca. 60 Gew.-% TDA |
| B | Wasserabführung |
| C | TDA/Wasser-Gemisch Rückführung zu A |
| D | TDA (Trocken-)Abführung Bodenzahl der Kolonne: 25 |

**Fig. 2:** Erfindungsgemäßes Verfahren
Es bedeuten:

| | |
|---|---|
| A | Zufuhr DNT |
| B | Zufuhr Wasserstoff |
| C | Abluft |
| D | Wärmeabfuhr |
| E | katalytische Umsetzung des DNT zum TDA (185°C, 25-30 bar, 2-3 Gew.-% Katalysator) |
| F | Katalysatorabtrennung |
| G | Entspannung auf 130°C, 3 bar |
| H | Zufuhr in Kolonne 1 |
| I | Wasserabführung (TDA-Gehalt: 25 ppm) |
| J | Abführung des erfindungsgemäßen TDA-Gemisches |
| K | Zwischenlagerung und Transport des erfindungsgemäßen TDA-Gemisches bei 80°C |
| L | Abführung TDA aus Kolonne 2 (Wassergehalt: 400 ppm) |
| M | Wasserabführung aus Kolonne 2 (TDA-Gehalt: 10 ppm) Bodenzahl Kolonne 1: 5 Bodenzahl Kolonne 2:10 |

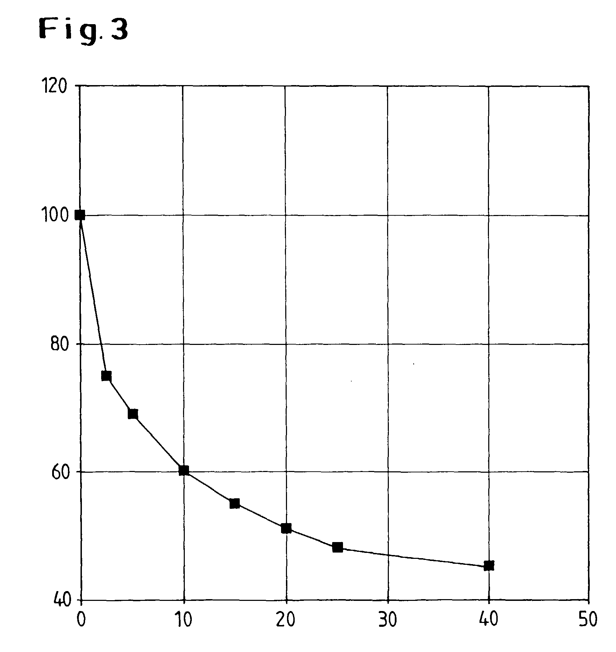

**Fig. 3:** Erstarrungstemperaturen von TDA/Wasser-Gemischen
(80 Gew.-% 2.4-TDA/20 Gew.-% 2.6-TDA)

| | |
|---|---|
| x-Achse | Wassergehalt in Gew.-% |
| y-Achse | Erstarrungstemperatur in °C. |

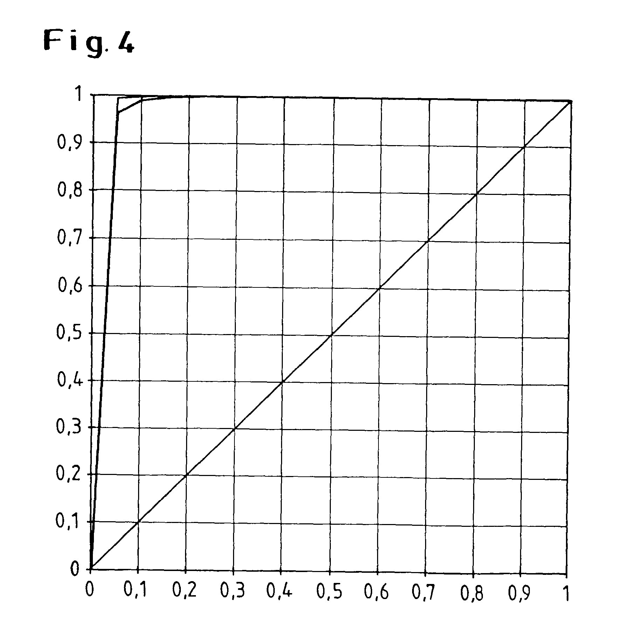

**Fig. 4:** Phasengleichgewicht Wasser/TDA
- 1013 mbar - 100 mbar

| | |
|---|---|
| x-Achse | Molanteil Wasser in der Flüssigphase |
| y-Achse | Molanteil Wasser in der Dampfphase. |

## Patentansprüche

1. Gemische aus Toluylendiamin und Wasser, die auf 100 Gew.-Teile Toluylendiamin 1 bis 40 Gew.-Teile Wasser enthalten und einen Gehalt an organischen, homogen gelösten Lösungs- oder Verdünnungsmitteln von maximal 10 Gew.-% aufweisen.

2. Gemische nach Anspruch 1, bei denen es sich bei den organischen, homogen gelösten Lösungs- oder Verdünnungsmitteln um Methanol, Ethanol, n-Propanol oder Isopropanol handelt.

3. Gemische nach Anspruch 1, bei denen es sich bei den organischen, homogen gelösten Lösungs- oder Verdünnungsmitteln um Aceton handelt.

4. Gemische nach Anspruch 1, bei denen es sich bei den organischen, homogen gelösten Lösungs- oder Verdünnungsmitteln um Diole handelt.

5. Gemische nach Anspruch 1, bei denen es sich bei den organischen, homogen gelösten Lösungs- oder Verdünnungsmitteln um Ethylenglykol handelt.

6. Gemische nach Anspruch 1, bei denen es sich bei den organischen, homogen gelösten Lösungs- oder Verdünnungsmitteln um Toluol handelt.

7. Gemische nach einem der Ansprüche 1 bis 6, die zusätzlich 0,05 bis 5 Gew.-% an 2,3- und/oder 3,4 Toluylendiamin und/oder 0,01 bis 2,5 Gew.-% an höhermolekularen Nebenprodukten enthalten.
